# EUROPÄISCHE PATENTSCHRIFT

(11) **EP 2 990 014 B1**
(45) Veröffentlichungstag und Bekanntmachung des Hinweises auf die Patenterteilung: **29.08.2018**
(21) Anmeldenummer: 15186165.5
(22) Anmeldetag: 27.01.2009
(51) Int. Cl.: A61F 2/66, A61F 2/68, A61F 2/50, A61F 2/70, A61F 2/74, A61F 2/76, A61F 5/01

(54) **PASSIVES ORTHOPÄDISCHES HILFSMITTEL IN FORM EINER FUSSPROTHESE ODER FUSSORTHESE**
PASSIVE ORTHOPAEDIC AID IN THE FORM OF A FOOT PROSTHETIC OR ORTHOTIC
PRODUIT D'AIDE ORTHOPEDIQUE PASSIF SOUS LA FORME D'UNE PROTHESE DE PIED OU ORTHESE DE PIED

(30) Priorität: 07.02.2008 DE 102008008281
(43) Veröffentlichungstag der Anmeldung: 02.03.2016
(62) Teilanmeldung aus: 09001076.0
(73) Patentinhaber: Ottobock SE & Co. KGaA, 37115 Duderstadt (DE)
(72) Erfinder: Mosler, Lüder, 37115 Duderstadt (DE); Pawlik, Roland, 1130 Wien (AT); Schörg, Joachim, 2353 Guntramsdorf (AT); Kaltenborn, Sven, 37115 Duderstadt (DE); Zarling, Sven, 37115 Duderstadt (DE); Schneider, Greg, Minneapolis, MN Minnesota MN 55403 (US)
(74) Vertreter: Lins, Edgar

(56) Entgegenhaltungen:
- WO-A-2006/000211
- WO-A-2006/112774
- WO-A-2008/103917
- US-A1- 2002 138 153
- US-A1- 2005 197 717
- US-A1- 2006 235 544

## Beschreibung

Die Erfindung betrifft ein passives orthopädisches Hilfsmittel in Form einer Fußprothese oder Fußorthese mit einem ersten Teil, das drehbar über ein Drehgelenk mit einem zweiten Teil verbunden ist, mit einer Sensoranordnung zur Messung von Parametern, die Rückschlüsse auf momentane Einsatzerfordernisse des Hilfsmittels erlauben, mit einer Prozessoranordnung zur Bestimmung von Einsatzerfordernissen und Erstellung entsprechender Steuersignale, mit einer steuerbaren hydraulischen Dämpferanordnung, mit der ein auf die Drehbewegung zwischen dem ersten Teil und dem zweiten Teil einwirkender Bewegungswiderstand veränderbar ist und mit einer die Steuersignale der Prozessoranordnung umsetzenden Steuerung zur Steuerung der Dämpferanordnung.

Derartige orthopädische Hilfsmittel sind insbesondere als Fußprothesen bekannt. Das Drehgelenk ist regelmäßig ein Knöchelgelenk, das ein Anschlussteil für eine Unterschenkelprothese mit einem Fußteil verbindet.

Bei einer aus US 2005/0197717 A1 bekannten Fußprothese weist das Fußteil eine elastische Sohlenfeder auf, die sich über die Länge des Fußteils erstreckt. Die durch die Elastizität der Sohlenfeder bewirkte Dämpfung in der Standphase des Gangzyklus wird dabei ergänzt durch eine aktive Verstellung des Winkels des Fußteils relativ zu dem Anschlussteil zum Unterschenkel. Dadurch lässt sich eine Anpassung an einen geneigten Untergrund oder an eine geänderte Absatzhöhe eines getragenen Schuhs realisieren, wobei für die Änderung der Absatzhöhe eine entsprechende manuelle Eingabe in die Steuerung vorgesehen ist. Der verwendete Aktuator ist vorzugsweise ein doppelt wirkender Motor, insbesondere in Form eines Doppelschraubenmotors. Die Verwendung eines doppelt aktiven Aktuators führt zu einem erheblichen Energiebedarf für die Funktion der Prothese, sodass entweder die Verwendung eines großvolumigen Akkumulators mit einer hohen Speicherkapazität oder ein häufiges Nachladen des Akkumulators vorgesehen werden muss.

In der nicht vorveröffentlichten Anmeldung WO 2008/103917 A1 ist eine Fußprothese beschrieben, bei der eine hydraulische Dämpfung dadurch erreicht wird, dass die Bewegung des Knöchelgelenks eine Bewegung eines Kolbens in einem Zylinder steuert, wodurch Hydraulikflüssigkeit aus einer Zylinderkammer in die andere Zylinderkammer gedrückt wird. Für beide Bewegungsrichtungen des Gelenks sind separate Verbindungsleitungen vorgesehen, in denen der Strömungswiderstand durch einstellbare Ventile separat einstellbar ist.

WO 2006/112774 A1 offenbart eine Steuerung für Gelenke einer Beinprothese, bei der von einer passiven Bedämpfung auf einen aktiven Antrieb des Gelenks mehrfach umgeschaltet wird. Dieser Steuerung liegt der Gedanke zu Grunde, dass eine rein passive Steuerung für die Steuerung des Gangzyklus zumindest in schwierigem Gelände nicht ausreichend sein könnte. Die passive Bedämpfung der Bewegung eines Gelenks einer Beinprothese erfolgt dabei über eine einzige Verbindungsleitung zwischen den Zylinderkammern.

Eine passive Fußprothese der eingangs erwähnten Art ist durch US 6,443,993 B1 bzw. US 2002/0138153 A1 bekannt. Zwischen einem Fußteil und einem Ansatzteil für einen Unterschenkel sind anterior und posterior vom Knöchelgelenk jeweils ein Dämpfungszylinder vorgesehen, die in einer Tandemanordnung funktionieren und mit einer gemeinsamen Verbindungsleitung miteinander verbunden sind. Bei Belastung der Ferse wird der posteriore Zylinder zusammengedrückt, sodass Hydraulikflüssigkeit durch die Verbindungsleitung in den anterioren Zylinder gelangt. Die Geschwindigkeit der Strömung der Hydraulikflüssigkeit durch die Verbindungsleitung - und damit die Dämpfung der entsprechenden Kompressionsbewegung an der Ferse - wird dadurch eingestellt, dass als hydraulische Flüssigkeit eine magneto-rheologische Flüssigkeit verwendet wird und mit einer Spule ein entsprechendes magnetisches Feld aufgebaut wird, das die Viskosität der magneto-rheologischen Flüssigkeit verändert. Als Sensoren werden dabei ein Absolut-Neigungssensor und ein Bodenkontaktsensor verwendet. Vorgesehen ist dabei, die Dämpfung in der Standphase des Gangzyklus beispielsweise beim durch den Neigungssensor festgestellten Überschreiten der Lotrechten, zu erhöhen, insbesondere von einem ersten Dämpfungspegel auf einen zweiten Dämpfungspegel umzuschalten. Dadurch ist eine gewisse Anpassung der Prothese an Untergrundschrägen und unterschiedliche Absatzhöhen möglich.

Die sich aus dem Stand der Technik ergebende Problemstellung besteht darin, dass die Steuerung einer passiven Fußprothese oder Fußorthese zwar einzelne Anpassungen ermöglicht, gegenüber dem Verhalten eines natürlichen, gesunden Fußes jedoch noch erhebliche Defizite aufweist.

Der vorliegenden Erfindung liegt daher die Aufgabe zugrunde, eine verbesserte Anpassung an das Verhalten eines natürlichen Fußes durch ein passives orthopädisches Hilfsmittel in Form einer Fußprothese oder Fußorthese zu ermöglichen.

Zur Lösung dieser Aufgabe ist erfindungsgemäß ein passives orthopädisches Hilfsmittel in Form einer Fußprothese oder Fußorthese der eingangs erwähnten Art dadurch gekennzeichnet, dass die Dämpferanordnung ein doppeltwirkender Hydraulikzylinder mit zwei durch einen Kolben voneinander getrennten Hydraulikkammern ist, dass die Hydraulikkammern über zwei, eine Strömung der Hydraulikflüssigkeit nur in entgegengesetzten Richtungen zulassenden Verbindungsleitungen verbunden sind, deren Strömungswiderstände durch die Steuerung separat mit jeweils einem eigenen Stellmittel einstellbar sind, dass die Prozessoranordnung zur Bestimmung einer aktuellen Nullpunktstellung aus den gemessenen Parametern der Sensoranordnung ausgelegt ist und dass die Einstellung der Steuersignale für die Strömungswiderstände in mindestens einer Verbindungsleitung ausgehend von der Nullpunktstellung erfolgt.

Die erfindungsgemäße Fußprothese oder Fußorthese weist somit einen doppelt wirkenden Hydraulikzylinder auf, dessen sich synchron zueinander verändernde Hydraulikkammern über zwei Verbindungsleitungen miteinander verbunden sind, wobei beide Verbindungsleitungen mit eigenen Stellmitteln zur Einstellung ihrer Strömungswiderstände versehen sind. Die Stellmittel können dabei insbesondere steuerbare Ventile sein, deren Durchflussquerschnitt - vorzugsweise stufenlos - veränderbar ist. Diese Anordnung bietet die Möglichkeit, während eines Gangzyklus unterschiedliche Strömungswiderstände für den Fluss der Hydraulikflüssigkeit in der einen und in der anderen Richtung schnell und präzise zu realisieren. Darüber hinaus kann die Verwendung der eigenen Stellmittel dazu ausgenutzt werden, einen stetigen Übergang von der Bewegung des Fußteils in die eine Richtung und in die andere Richtung zu gewährleisten. So ist es beispielsweise für die Steuerung des Knöchelgelenks möglich, einen kontrollierten Übergang von der Plantarflexion in die Dorsalflexion beim Übergang der Gewichtsbelastung von der Ferse auf die Fußspitze zu realisieren, andererseits aber bei der Entlastung der Fußspitze beim Übergang von der Standphase in die Schwungphase des Gangzyklus die ausgebildete Dorsalflexion für eine gewisse Zeit beizubehalten, um das Anheben der Fußspitze am Beginn der Schwungphase bis zum Durchschwingen des Beines über eine Mittel-position hinaus beizubehalten, wie dies mit einem natürlichen gesunden Fuß erfolgt, um ein Anstoßen der Zehenspitzen auf dem Boden beim Durchschwingen zu vermeiden.

Erfindungsgemäß sollen die Sensoranordnung und die Prozessoranordnung so ausgelegt sein, dass jeweils eine aktuelle Nullpunktstellung bestimmbar ist. Eine hierfür geeignete Prozessoranordnung besteht beispielsweise aus einem Knöchelmomentsensor und einem Neigungs- bzw. Absolutwinkelsensor, wenn das Drehgelenk die Funktion eines Knöchelgelenks ausübt. Ergänzend hierzu kann vorzugsweise ein Knöchelwinkelsensor vorgesehen sein. Die Bestimmung eines aktuellen Anschlags nach dorsal ist dabei durch die Ermittlung des Absolutwinkels beim Nulldurchgang des Knöchelmoments in der Standphase des Gangzyklus möglich. Dadurch kann die aktuelle Untergrundneigung bzw. die jeweilige Absatzhöhe bei jedem Gangzyklus ohne Zeitverzögerung berücksichtigt werden. Da dabei der Einfluss der Absatzhöhe und der Untergrundneigung in gleicher Weise in das gemessene Signal eingehen, ist es zweckmäßig, den konstanten Einfluss der Absatzhöhe durch eine entsprechende Auswertung der Signale in der Standphase des Gangzyklus oder beim Stehen zu ermitteln. Alternativ hierzu kann die Absatzhöhe auch manuell in die Auswertungseinrichtung eingebbar sein.

Bei einer bevorzugten Ausführungsform der Erfindung können die Strömungswiderstände in den beiden Verbindungsleitungen so groß eingestellt werden, dass das Drehgelenk in einer Stellung arretiert ist. Für die Arretierung des Drehgelenks können daher die Stellmittel verwendet werden, sodass keine gesonderte Arretierungseinrichtung erforderlich ist, beispielsweise um beim Stehen des Trägers des orthopädischen Hilfsmittels eine stabile Unterstützung durch die Prothese oder Orthese zu gewährleisten. Die arretierte Stellung kann dabei der ermittelten Nullpunktstellung entsprechen.

In einer besonders bevorzugten Ausführungsform der Erfindung sind die Teile des Hilfsmittels überwiegend starr ausgebildet. Die Steuerung wenigstens eines überwiegenden Teils des Bewegungsablaufes innerhalb eines Bewegungszyklus erfolgt ohne Einfluss von Materialelastizitäten durch die Steuerung der Strömungswiderstände. Allenfalls kann es zweckmäßig sein, eine gewisse Materialelastizität beim Aufsetzen der Ferse auf den Untergrund beim Gehen auszunutzen.

Mit dem erfindungsgemäßen orthopädischen Hilfsmittel wird somit eine Nullpunktstellung der Drehbewegung bestimmt, von der aus bei einem zyklischen Bewegungsablauf die Bewegungswiderstände für die erste und die zweite Bewegungsrichtung des Drehgelenks, insbesondere des Knöchelgelenks, bestimmt werden. Für ein definiertes Einsatzerfordernis, beispielsweise beim Stehen, wird durch die Einstellung hoher Bewegungswiderstände in beiden Bewegungsrichtungen das Drehgelenk arretiert, vorzugsweise in dem Nullpunkt. Der Bewegungszyklus ist dabei vorzugsweise ein Gangzyklus.

In einer weiter bevorzugten Ausführungsform der Erfindung ist das am Drehgelenk angeschlossene Teil ein Fußteil, das in ein Hauptfußteil und ein Vorderfußteil unterteilt ist, wobei das Vorderfußteil mit dem Hauptfußteil über ein Gelenk verbunden ist. Der Hydraulikzylinder kann dabei an dem Vorderfußteil angelenkt sein, um so das Hauptfußteil in seiner Winkelstellung indirekt, nämlich über das Vorderfußteil, einzustellen.

Die Erfindung soll im Folgenden anhand eines in der Zeichnung dargestellten Ausführungsbeispiels näher erläutert werden. Es zeigen:
- Figur 1: eine schematische Darstellung eines ersten Ausführungsbeispiels einer erfindungsgemäßen Fußprothese;
- Figur 2: eine schematische Darstellung eines zweiten Ausführungsbeispiels einer erfindungsgemäßen Fußprothese;
- Figur 3: eine schematische Darstellung eines dritten Ausführungsbeispiels einer erfindungsgemäßen Fußprothese;
- Figur 4: eine Draufsicht auf eine konstruktiv detaillierte weitere Ausführungsform einer erfindungsgemäßen Fußprothese;
- Figur 5: einen Vertikalschnitt parallel zur Sagittalebene durch die Fußprothese gemäß Figur 4.

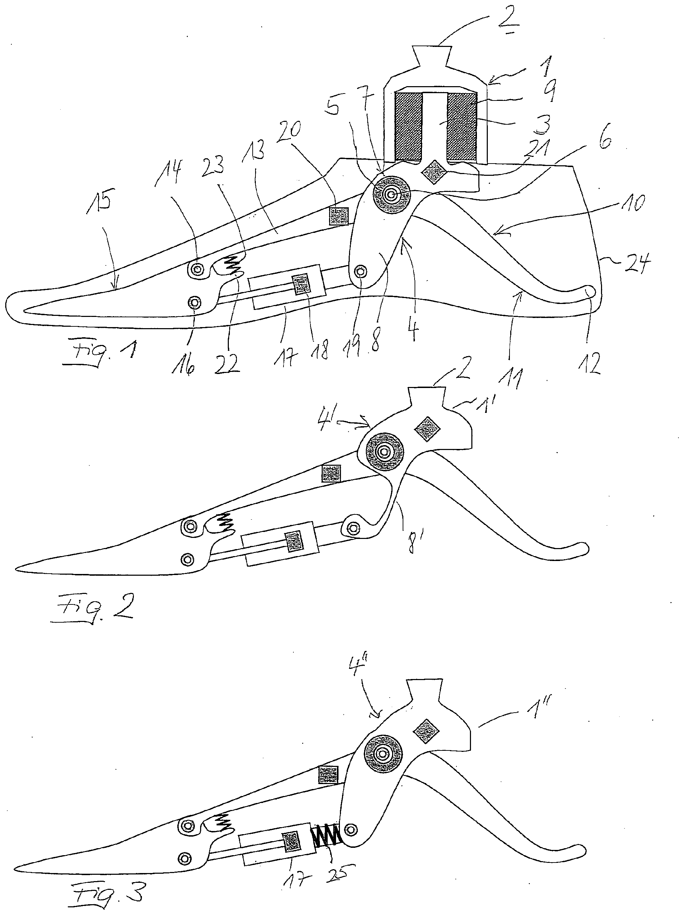

Bei dem in Figur 1 dargestellten Ausführungsbeispiel ist ein Befestigungsstück 1 mit einem Justieransatz 2 in Form eines umgekehrten Pyramidenstumpfes mit vier Schrägflächen ausgebildet. Das Befestigungsstück 1 bildet einen nach unten offenen Topf, in den ein nach oben ragender Steg 3 eines zweiarmigen Hebels 4 hineinragt. Der zweiarmige Hebel ist um ein Drehgelenk 5 drehbar, dessen Drehachse 6 zugleich die Achse eines Knöchelgelenks des künstlichen Fußes bildet. Das Drehgelenk 5 ist mit einem Winkelsensor 7 versehen. Der zweiarmige Hebel 4 weist einen starren, nach unten ragenden Ansatz 8 auf.

In dem nach unten offenen Topf des Befestigungsstücks 1 ist der mit dem Steg 3 gebildete Zwischenraum durch ein relativ steifes, elastisches Material 9 ausgefüllt, sodass die Bewegung des Befestigungsstücks 1 nur etwas bedämpft auf Bewegung des Stegs 3 des zweiarmigen Hebels 4 übertragen wird. Demgemäß vollzieht der Ansatz 8 die Bewegung des Befestigungsstücks 1 nach, jedoch aufgrund des elastischen Materials 9 etwas bedämpft.

Das das Knöchelgelenk bildende Drehgelenk 5 trägt ferner ein Hauptfußteil 10, das sich ebenfalls als zweiarmiger Hebel mit einem hinteren Hebelarm 11 in den Fersenbereich des Fußes hinein erstreckt, wo der schräg nach hinten und unten verlaufende hintere Hebelarm 11 mit einem etwa horizontal abgerundeten Ende 12 versehen ist.

Das Hauptfußteil weist einen vom Knöchelgelenk 5 nach vorn erstreckten vorderen Hebelarm 13 auf, der sich nahezu geradlinig vom Knöchelgelenk 5 nach vorn etwas schräg nach unten verlaufend erstreckt, sodass das Hauptfußteil 10 zum Knöchelgelenk 5 hin nach oben gewölbt ausgebildet ist und vom Knöchelgelenk 5 nach hinten in den Fersenbereich sowie nach vorne in einen Vorderfußbereich hinein schräg abfällt, wobei der schräge Abfall in den Fersenbereich hinein steiler ist als in den Vorderfußbereich.

Der vordere Hebelarm 13 des Hauptfußteils 10 endet am Beginn des Vorderfußbereichs und trägt dort ein Drehgelenk 14, mit dem ein einen Zehenbereich nachbildendes Vorderfußteil 15 drehbar an dem vorderen Hebelarm 13 des Hauptfußteils angelenkt ist. Das Drehgelenk 14 weist eine Drehachse auf, die parallel zur Drehachse 6 des Knöchelgelenks 5 in horizontaler Richtung verläuft. Da das Vorderfußteil 15 den Zehenbereich eines natürlichen Fußes imitiert, ist es nach vorne hin dreieckförmig mit einer Spitze auslaufend ausgebildet. Unterhalb des Drehgelenks 14 befindet sich am Vorderfußteil 15 ein weiteres Drehgelenk 16, mit dem eine Kolbenstange eines Kolbens 18 eines Hydraulikzylinders 17 an dem Vorderfußteil 15 angelenkt ist. Der Hydraulikzylinder 17 ist mit einem Drehgelenk 19 drehbar an dem freien Ende des nach unten ragenden Ansatzes 8 des zweiarmigen Hebels 4 angelenkt, sodass das Drehgelenk 19 unterhalb und etwas nach vorn (in Richtung Vorderfußbereich 15) versetzt zum Knöchelgelenk 5 angeordnet ist.

Das Knöchelgelenk 5 enthält den Winkelsensor 7 für die Messung des Knöchelwinkels, also des Winkels zwischen dem (fluchtend mit dem Unterschenkel ausgerichteten) Steg 3 und dem vorderen Hebelarm 13 des Hauptfußteils 10.

Der vordere Hebelarm 13 des Hauptfußteils 10 trägt ferner einen Neigungssensor 20, der die Neigung relativ zur Erdanziehungskraft (relativ zur Lotrechten) ermittelt. Derartige Neigungssensoren 20, die einen absoluten Neigungswinkel relativ zur Erdbeschleunigung bestimmen, sind als Beschleunigungssensoranordnungen mit oder ohne Gyroskop bekannt.

Der zweiarmige Hebel 4 enthält fluchtend mit dem Justieransatz 2, d.h. fluchtend mit dem (künstlichen) Unterschenkel des Patienten einen Knöchelmomentsensor 21, der das an dieser Stelle wirkende Drehmoment misst.

Das Vorderfußteil 15 ist an seinem hinteren Ende mit einem Lageransatz 22 versehen, der zum Halten einer auf Zug und Druck belastbaren Feder 23 dient, die sich mit ihrem anderen Ende am vorderen Hebelarm 13 des Hauptfußteils 10 abstützt. Die Feder 23 bewirkt eine Rückstellung des Vorderfußteils 15 nach einer Dorsalflexion, wobei die Rückstellgeschwindigkeit durch den Hydraulikzylinder 17 bestimmt wird.

Der Hydraulikzylinder 17 ist als passiver Aktuator ausgebildet, in dem der vom Kolben 18 bewirkte Hydraulikfluss durch (nicht dargestellte) Ventile gesteuert wird, wobei die Ventile selbst nicht nur ein- und ausschaltbar sind, sondern auch für eine definierte Durchflussmenge gesteuert werden können.

Die in Figur 2 dargestellte Ausführungsform entspricht im Wesentlichen der Ausführungsform gemäß Figur 1. Ein Unterschied besteht darin, dass das Befestigungsstück 1' mit dem Justieransatz 2 einstückig ausgebildet ist, sodass eine durch das elastische Material 9 gebildete Elastizität nicht mehr vorhanden ist. Stattdessen ist der nach unten ragende Ansatz 8' des zweiarmigen Hebels 4' mit einer Materialverdünnung ausgebildet, sodass das das Drehgelenk 19 tragende freie Ende des Ansatzes elastisch federnd zum restlichen Material des zweiarmigen Hebels 4' angeordnet ist.

Selbstverständlich weist der künstliche Fuß gemäß der zweiten Ausführungsform ebenso einen kosmetischen Überzug 24 auf, wie die erste Ausführungsform. Dieser kosmetische Überzug 24 ist für die zweite und dritte Ausführungsform jedoch nicht wiederholt dargestellt.

Bei dem künstlichen Fuß gemäß der in Figur 3 dargestellten dritten Ausführungsform ist der zweiarmige Hebel 4" ebenfalls einstückig mit dem Justieransatz 2 ausgebildet. Auch der nach unten ragende Ansatz 8 des zweiarmigen Hebels 4" ist starr wie bei der ersten Ausführungsform. Stattdessen ist der Hydraulikzylinder 17 über eine Spiralfeder 25 elastisch mit dem nach unten ragenden Ansatz 8 des zweiarmigen Hebels 4" verbunden. Dadurch wird in Serie mit der Wirkung des Hydraulikzylinders 17 eine Elastizität verwirklicht, die bei der in Figur 1 dargestellten Ausführungsform durch das elastische Material 9 und bei der in Figur 2 dargestellten Ausführungsform durch den federnden Ansatz 8' realisiert ist. Alle übrigen Teile der dritten Ausführungsform entsprechen denen der ersten Ausführungsform.

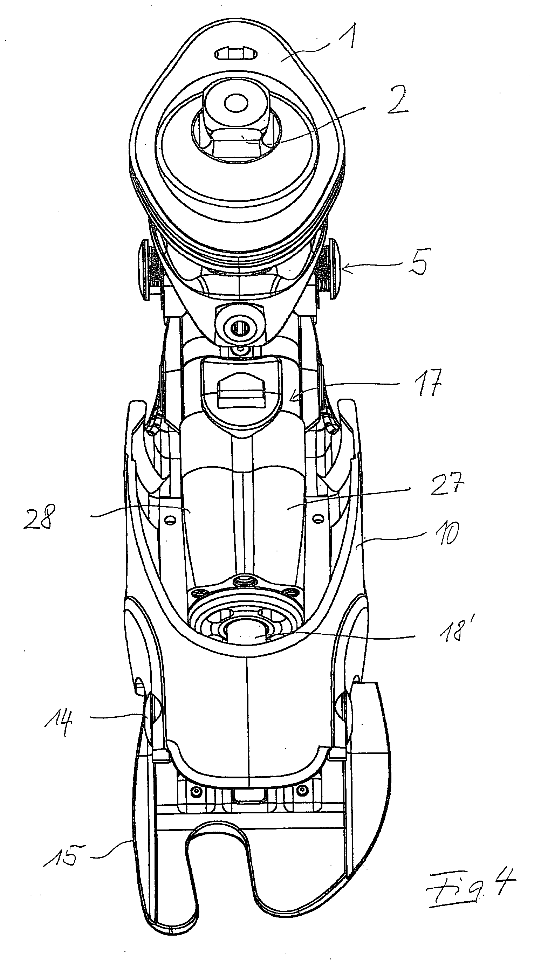

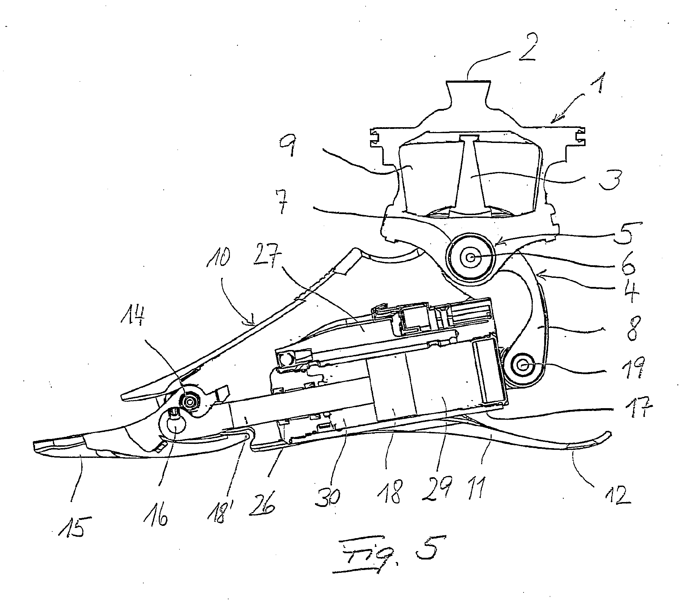

Das in den Figuren 4 und 5 dargestellte Ausführungsbeispiel lässt das Befestigungsstück 1 mit dem pyramidenförmigen Justieransatz 2 erkennen. In dem Befestigungsstück 1 befindet sich das elastische Material 9, das mit dem nach oben ragenden Steg 3 des zweiarmigen Hebels 4 dämpfend zusammenwirkt. Der nach unten ragende Ansatz 8 des zweiarmigen Hebels 4 verläuft in diesem Ausführungsbeispiel in Gehrichtung hinter dem Knöchelgelenk 5 und ist dort über das Drehgelenk 19 an dem Hydraulikzylinder 17 angelenkt. In dem Hydraulikzylinder 17 bewegt sich längsverschiebbar der Kolben 18, der über ein im Hydraulikzylinder 17 befindliches Lager 26 herausgeführt und mit dem weiteren Drehgelenk 16 des Vorderfußteils 15 verbunden ist. Das Knöchelgelenk 5 lagert ferner das Hauptfußteil 10, das hier in Form eines starren Gehäuses ausgebildet ist und einen nach hinten gerichteten elastischen Hebel 11 als Fersenhebel aufweist. Das Fußteil 10 ist mit dem Fersenhebel 11 somit gemeinsam um das Knöchelgelenk 5 relativ zu dem Befestigungsstück 1 und zu dem zweiarmigen Hebel 4 schwenkbar. Die Schwenkbewegung zwischen dem Befestigungsstück 1 und dem Fußteil 10 wird über den zweiarmigen Hebel 4 und den Hydraulikzylinder 17 gesteuert und bedämpft. Die Anlenkung der Kolbenstange 18' des Kolbens 18 an dem Vorderfußteil 15 bewirkt dabei lediglich eine zusätzliche Steuerung des die Zehenplatte bildenden Vorderfußteils 15, die jedoch die Steuerung des Hauptfußteils 10 nur geringfügig modifiziert, da das weitere Drehgelenk 16 in unmittelbarer Nähe des Drehgelenks 14 zwischen dem Vorderfußteil 15 und dem Hauptfußteil 10 angeordnet ist. Der Hydraulikzylinder 17 ist mit zwei Steuerventilen 27, 28 versehen, die auf der Oberseite des Hydraulikzylinders 17 angeordnet sind. Die Steuerventile 27, 28 sind mit Kammern 29, 30 des Hydraulikzylinders 17 beiderseits des Kolbens 18 verbunden, wobei (nicht dargestellte) Rückschlagventile dafür sorgen, dass durch das erste Steuerventil 27 lediglich ein Fluss der Hydraulikflüssigkeit von der hinteren Kammer 29 zur vorderen Kammer 30 erfolgen kann, wodurch die Einschubbewegung des Kolbens 18 in den Hydraulikzylinder 17 ermöglicht wird, was einer Plantarflexion des Hauptfußteils 10 gegenüber dem Befestigungsstück 1 entspricht. Der andere Steuerzylinder 28 ermöglicht mit Rückschlagventilen lediglich den Hydraulikfluss von der vorderen Kammer 30 in die hintere Kammer 29, wodurch ein Herausziehen des Kolbens 18 aus dem Hydraulikzylinder 17 ermöglicht wird, also eine Verlängerung des Abstandes zwischen den Drehgelenken 19, 16 stattfindet. Dies entspricht einer Dorsalflexion zwischen dem Befestigungsstück 1 und dem Hauptfußteil 10. Gleichzeitig wird durch die Verschiebung des Drehgelenks 16 gegenüber dem Drehgelenk 14 nach vorn ein Anheben des Vorderfußteils 15 bewirkt.

Die Funktion des künstlichen Fußes ist für die dargestellten Ausführungsformen gleich. Mit der Sensoranordnung zur Messung des Knöchelwinkels, des Knöchelmoments und des absoluten Neigungswinkels lassen sich die relevanten Funktionszustände des künstlichen Fußes ermitteln und differenzieren, wobei das Signal des Knöchelwinkelsensors zur Ermittlung des Knöchelwinkels (zwischen Befestigungsstück 1, 1', 1" und Hauptfußteil 10) einerseits und die jeweilige Knöchelwinkelgeschwindigkeit andererseits ausgewertet werden.

So kann beispielsweise die Erkennung, ob mit dem künstlichen Fuß gegangen wird oder gestanden wird, dadurch erfolgen, dass die Knöchelwinkelgeschwindigkeit im Nulldurchgang des Knöchelmoments bestimmt wird. Liegt die Knöchelwinkelgeschwindigkeit im Nulldurchgang des Knöchelmoments unter einem Schwellwert, wird dies als "Stehen" erkannt und der Aktuator in Form des Hydraulikzylinders 17 mittels der Ventile auf einen hohen Widerstand eingestellt, durch den ein Dorsalanschlag gebildet werden kann.

Die Erkennung einer abschüssigen Neigung oder der Absatzhöhe wird durch den Neigungssensor 20 im Mittelfußbereich des Hauptfußteils 10 beim Nulldurchgang des Knöchelmoments bestimmt.

Wird das Gehen in der Ebene detektiert, wird das Ventil, das für die Plantarflexion des Fußes zuständig ist, in einer halboffenen Stellung belassen, während das Ventil, das die Dorsalflexion bestimmt, mit zunehmenden Knöchelwinkel geschlossen wird, um einen Dorsalanschlag zu bilden.

Wird ein Gehen bergauf detektiert, wird dabei eine erweiterte Dorsalflexion des Vorderfußteils 15 zugelassen.

Wird beim Gehen der Fersenauftritt nach der Schwungphase und zu Beginn der Standphase insbesondere durch ein negatives Knöchelmoment detektiert, wird das Ventil für die Plantarflexion so gesteuert, dass es mit zunehmenden Knöchelwinkel in Richtung der Plantarflexion schließt und somit einen Anschlag für die Plantarflexion bildet.

Wird ein Zehenabstoß am Ende der Standphase detektiert (abfallendes Knöchelmoment bei vergrößertem Knöchelwinkel), wird das Ventil für die
Dorsalflexion nach einer Totzeit vollständig geöffnet, um durch ein elastisches Element die Anhebung des Vorderfußteils (Zehenanhebung) in der Schwungphase auszulösen.

An diesen Beispielen ist erkennbar, dass die wichtigen Steuerungen eines künstlichen Fußes beim Stehen und beim Gehen auch in Abhängigkeit von der Bodenneigung oder der Absatzhöhe zutreffend vorgenommen werden können, wobei die Steuerung des Bewegungswiderstandes über den Hydraulikzylinder bereits ausreicht.

In einem Ausführungsbeispiel für die Erkennung der Bewegungszustände für die Fußprothese und die daraus resultierende Steuerung werden folgende Funktionen realisiert:

### Unterscheidung Stehen-Gehen

Die Unterscheidung zwischen Stehen und Gehen erfolgt mit folgenden Kriterien:

### 1. Erkennung einer Schwungphase

Eine Schwungphase wird dadurch erkannt, dass das Knöchelmoment nahe Null ist, da der Fuß in der Schwungphase unbelastet ist.

Der Absolutwinkel des Unterschenkels überschreitet einen Schwellenwert, der für das Stehen individuell definiert sein kann. Ferner überschreitet die Absolutwinkelgeschwindigkeit einen definierten Schwellenwert.

### 2. Erkennen eines Fersenauftritts im vorgeschwungenen Zustand

Es wird ein negatives Knöchelmoment (plantarflektierend) detektiert. Das Absolutwinkelsignal entspricht einem vorgeschwungenen Fuß im Vergleich zu einem für das Stehen individuell definierten Schwellenwert.

Optional kann eine Plantarflexion beim Fersenauftritt über die Knöchelwinkelgeschwindigkeit angezeigt werden.

### 3. Rückkehr zum Stehen

Nach einem detektierten Fersenauftritt verbleibt der Absolutwinkel des Unterschenkels innerhalb eines für das Stehen individuell definierten Schwellenwertes. Alternativ oder ergänzend kann eine aktive Umkehr der Bewegungsrichtung in der mittleren Standphase von dorsal nach plantar als Kriterium für das Stehen erkannt werden.

Beim detektierten Stehen werden die Steuerventile 27, 28 so eingestellt, dass sich für das Stehen Anschläge nach ventral und dorsal für einen engen Winkel (Nullpunktlage) ergeben. Für den Gangzyklus wird der Anschlag nach dorsal verschoben und die Dämpfungseigenschaften für die Plantar- und Dorsalflexion in Abhängigkeit von der Schrittlänge eingestellt.

### Unterscheidung Ebene-Rampe

Der gemessene Absolutwinkel zu Beginn der mittleren Standphase im Gehzyklus, also nach dem Aufsetzen des vollen Fußes auf den Untergrund, ist größer oder kleiner als ein für das Gehen in der Ebene definierter Wertebereich für den Absolutwinkel.

Entsprechend der festgestellten Neigung der Rampe wird der Dorsalanschlag verändert und die Dämpfungseigenschaften bei der Plantarflexion und der Dorsalflexion werden in Abhängigkeit des Absolutwinkels und der prognostizierten Schrittlänge eingestellt.

### Erkennung Rückwärtsgehen

Die Erkennung des Rückwärtsgehens besteht aus einer Erkennung der Rückschwungphase und der Erkennung eines Vorderfußauftritts in dem rückgeführten Zustand.

### 1. Erkennung einer Rückschwungphase

Bei einem gemessenen Knöchelmoment nahe Null entspricht das Absolutwinkelsignal einem gegenüber dem Stehen zurückgeführten Fuß (Retroversion) und die Absolutwinkelgeschwindigkeit überschreitet einen definierten Schwellenwert.

### 2. Erkennung des Vorderfußauftritts im rückgeführten Zustand

Gemessen wird ein größeres positives Knöchelmoment.

In Abhängigkeit von den gemessenen Werten wird der Anschlag nach dorsal verstellt und die Dämpfungseigenschaften bei Plantar- und Dorsalflexion werden in Abhängigkeit vom Absolutwinkel bei dem Vorfußauftritt eingestellt.

### Anpassung an unterschiedliche Absatzhöhen

Vorzugsweise wird die Absatzhöhe durch das Auslesen des Absolutwinkelsignals während eines manuell ausgelösten Triggersignals festgestellt. Proportional zum Absolutwinkel wird der Nullpunkt für die Steuerventile 27, 28 eingestellt.

Alternativ hierzu ist es möglich, die Absatzhöhe von einer Rampenneigung bei einem künstlichen Fuß mit einem gelenkig angeschlossenen Vorderfußteil 15 dadurch zu bestimmen, dass der Winkel des Vorderfußteils 15 in Relation zum Hauptfußteil 10 gemessen wird. Hierin liegt eine zusätzliche Option im Rahmen der vorliegenden Erfindung.

### Stehen auf geneigtem Untergrund

Gemessen wird der Absolutwinkel bei einer Umkehr der Bewegungsrichtung von plantar nach dorsal, wenn das Knöchelmoment einen Nulldurchgang erfährt.

Dementsprechend wird der dorsale Anschlag für die Steuerung des Hydraulikzylinders 17 mit den Steuerventilen 27, 28 in Abhängigkeit von der Untergrundneigung verstellt.

### Erkennung Treppengehen

Wenn der Absolutwinkelsensor 20 aus zwei Beschleunigungssensoren für senkrecht aufeinanderstehende Beschleunigungskomponenten besteht und die Beschleunigungskomponenten separat ausgebbar sind, kann der vertikal zurückgelegte Weg und der horizontal zurückgelegte Weg des Hauptfußteils 10 festgestellt werden. Die Wegstrecken werden durch zweifache Integration über die entsprechenden Beschleunigungskomponenten ermittelt. In diesen Fällen ist es möglich, eine Unterscheidung von Treppauf- und Treppabgehen vorzunehmen und dementsprechend die Anschläge für die Dämpfungseigenschaften bei Plantar- und Dorsalflexion einzustellen.

In ähnlicher Weise können die Beschleunigungen ausgenutzt werden, um das Gehen bei unterschiedlichen Ganggeschwindigkeiten, einzustellen, indem die Anschläge nach dorsal sowie die Dämpfungseigenschaften bei Plantar- und Dorsalflexion entsprechend geändert werden.

## Patentansprüche

1. Passives orthopädisches Hilfsmittel in Form einer Fußprothese oder Fußorthese mit einem Fußteil, das drehbar über ein Drehgelenk mit einem Anschlussteil für eine Unterschenkelprothese verbunden ist, mit einer Sensoranordnung zur Messung von Parametern, die Rückschlüsse auf momentane Einsatzerfordernisse des Hilfsmittels erlauben, mit einer mit der Sensoranordnung verbundenen Prozessoranordnung zur Bestimmung von Einsatzerfordernissen und Erstellung entsprechender Steuersignale, mit einer steuerbaren hydraulischen Dämpferanordnung, mit der ein auf die Drehbewegung zwischen dem ersten Teil und dem zweiten Teil einwirkender Bewegungswiderstand veränderbar ist und mit einer die Steuersignale der Prozessoranordnung umsetzenden Steuerung zur Steuerung der Dämpferanordnung, **dadurch gekennzeichnet, dass** die Dämpferanordnung ein doppelt wirkender Hydraulikzylinder mit zwei durch einen Kolben voneinander getrennten Hydraulikkammern ist und dass die Hydraulikkammern über zwei, eine Strömung der Hydraulikflüssigkeit nur in entgegengesetzten Richtungen zulassenden Verbindungsleitungen verbunden sind, deren Strömungswiderstände durch die Steuerung separat mit jeweils einem eigenen Stellmittel einstellbar sind, dass die Prozessoranordnung zur Bestimmung einer aktuellen Nullpunktstellung aus den gemessenen Parametern der Sensoranordnung ausgelegt ist und dass die Erstellung der Steuersignale für die Strömungswiderstände in den beiden Verbindungsleitungen in Bezug auf die Nullpunktstellung erfolgt, **dadurch gekennzeichnet, dass** das Hilfsmittel einen Neigungs- oder Absolutwinkelsensor zum Bestimmen eines Absolutwinkels der Unterschenkelprothese aufweist.

2. Orthopädietechnisches Hilfsmittel nach Anspruch 1, **dadurch gekennzeichnet, dass** der Strömungswiderstand in zumindest einer Verbindungsleitung so groß einstellbar ist, dass das Drehgelenk in einer beliebigen Stellung arretierbar ist.

3. Orthopädietechnisches Hilfsmittel nach Anspruch 2, **dadurch gekennzeichnet, dass** die arretierte Stellung der Nullpunktstellung entspricht.

4. Orthopädietechnisches Hilfsmittel nach einem der Ansprüche 1 bis 3, **dadurch gekennzeichnet, dass** die Nullpunktstellung in Abhängigkeit einer Neigung des Untergrunds bestimmbar ist.

5. Orthopädietechnisches Hilfsmittel nach Anspruch 4, **dadurch gekennzeichnet, dass** die Nullpunktstellung in Abhängigkeit von einer aktuellen Absatz-höhe eines mit dem Hilfsmittel verwendeten Schuhs bestimmbar ist.

6. Orthopädietechnisches Hilfsmittel nach einem der Ansprüche 1 bis 5, **dadurch gekennzeichnet, dass** die Teile des Hilfsmittels überwiegend starr ausgebildet sind und dass die Steuerung wenigstens eines überwiegenden Teils des Bewegungsablaufs innerhalb eines Bewegungszyklusses ohne Einfluss von Materialelastizitäten durch die Steuerung der Strömungswider-stände erfolgt.

7. Orthopädietechnisches Hilfsmittel nach einem der Ansprüche 1 bis 6, **dadurch gekennzeichnet, dass** das Drehgelenk ein Knöchelgelenk ist.

8. Verfahren zur Steuerung eines orthopädietechnisches Hilfsmittels nach einem der Ansprüche 1 bis 7, **dadurch gekennzeichnet, dass** eine Nullpunktstellung der Drehbewegung bestimmt wird, von der aus bei einem zyklischen Bewegungsablauf der Bewegungswiderstand für mindestens eine Bewegungsrichtung bestimmt wird.

9. Verfahren nach Anspruch 8, **dadurch gekennzeichnet, dass** das Hilfsmittel für ein definiertes Einsatzerfordernis durch Einstellung hoher Bewegungswiderstände in beiden Bewegungsrichtungen in dem Nullpunkt arretiert wird.

10. Verfahren nach Anspruch 7 oder 8, angewendet auf ein orthopädietechnisches Hilfsmittel für eine untere Extremität, bei der ein Gangzyklus als steuerbarer Bewegungszyklus benutzt wird.

## Claims

1. Passive orthopedic aid in the form of a foot prosthesis or foot orthosis, with a first part (4) which is connected to a second part (10) in a rotatable manner via a swivel joint (5), with a sensor arrangement (7, 20, 21) for measuring parameters that provide indications of instantaneous input requirements of the aid, with a control means which is connected to the control arrangement (27, 28) and is used to determine input requirements and to generate corresponding control signals, with a controllable hydraulic damping arrangement (17, 18) with which a movement resistance acting on the rotation movement between the first part and the second part can be modified, and with a control means which converts the control signals of the processor arrangement and is used to control the damping arrangement, wherein the damping arrangement is a dual-action hydraulic cylinder (17) with two hydraulic chambers (29, 30) separated from each other by a piston (18), and the hydraulic chambers are connected via two connection lines which permit a flow of the hydraulic fluid only in mutually opposite directions and whose flow resistances can be adjusted by the control separately and in each case via their own adjustment means, **characterized in that** the sensor arrangement (7, 20, 21) is configured for measuring an ankle moment of the swivel joint (5) and **in that** the processor arrangement is configured for determining a current neutral point position by evaluating the zero-crossing of the ankle moment and **in that** the control signals for the flow resistances in the two connection lines are generated with respect to the neutral point position.

2. Orthopedic aid according to Claim 1, **characterized in that** the flow resistance in at least one connection line can be made so great that the swivel joint (5) can be locked in any desired position.

3. Orthopedic aid according to Claim 2, **characterized in that** the locked position corresponds to the neutral point position.

4. Orthopedic aid according to one of Claims 1 to 3, **characterized in that** the neutral point position can be determined as a function of an inclination of the ground.

5. Orthopedic aid according to Claim 4, **characterized in that** the neutral point position can be determined as a function of a current height of the heel of a shoe that is used with the aid.

6. Orthopedic aid according to one of Claims 1 to 5, **characterized in that** the parts of the aid are mostly rigid, and **in that** at least most of the movement within a movement cycle is controlled by the control of the flow resistances, without being influenced by material elasticities.

7. Orthopedic aid according to one of Claims 1 to 6, **characterized in that** the swivel joint (5) is an ankle joint.

8. Method for controlling an orthopedic aid according to one of Claims 1 to 7, **characterized in that** a neutral point position of the rotation movement is determined from which, in a cyclical movement, the movement resistance for at least one movement direction is determined.

9. Method according to Claim 8, **characterized in that** the aid, for a defined input requirement, is locked in the neutral point by setting high movement resistances in both directions of movement.

10. Method according to Claim 7 or 8, applied to an orthopedic aid for a lower extremity, in which a gait cycle is used as controllable movement cycle.

## Revendications

1. Accessoire orthopédique passif sous la forme d'une prothèse ou d'une orthèse de pied comprenant une partie de pied, qui est reliée de manière rotative à une partie de raccordement pour une prothèse de membre inférieur par le biais d'une articulation rotative, comprenant un agencement capteur pour la mesure de paramètres qui permettent de faire des conclusions quant aux exigences d'emploi momentanées de l'accessoire, comprenant un agencement à processeur, relié à l'agencement capteur, pour la détermination des exigences d'emploi et la génération de signaux de commande correspondants, comprenant un agencement amortisseur hydraulique susceptible d'être commandé, au moyen duquel une résistance agissant sur le mouvement rotatif entre la première partie et la seconde partie peut être variée, et comprenant une commande, qui convertit les signaux de commande de l'agencement à processeur afin de commander l'agencement amortisseur, **caractérisé en ce que** l'agencement amortisseur comprend un cylindre hydraulique à double effet doté de deux chambres hydrauliques séparées l'une de l'autre par un piston, et **en ce que** les chambres hydrauliques sont reliées via deux conduits de liaison qui permettent un écoulement du liquide hydraulique uniquement dans des sens opposés, conduits dont les résistances à l'écoulement peuvent être réglées par la commande séparément à l'aide d'un organe de positionnement respectif propre, **en ce que** l'agencement à processeur est conçu pour déterminer une position de point zéro actuelle à partir des paramètres mesurés de l'agencement capteur, et **en ce que** la génération des signaux de commande pour les résistances d'écoulement a lieu dans les deux conduits de liaison par référence à la position de point zéro, **caractérisé en ce que** l'accessoire comprend un capteur d'inclinaison ou d'angle absolu servant à déterminer un angle absolu de la prothèse de membre inférieur.

2. Accessoire orthopédique selon la revendication 1, **caractérisé en ce que** la résistance à l'écoulement dans au moins un conduit de liaison est susceptible d'être réglée de manière suffisamment élevée pour que l'articulation rotative puisse être arrêtée dans une position quelconque.

3. Accessoire orthopédique selon la revendication 2, **caractérisé en ce que** la position arrêtée correspond à la position de point zéro.

4. Accessoire orthopédique selon l'une des revendications 1 à 3, **caractérisé en ce que** la position de point zéro est susceptible d'être déterminée en fonction d'une inclinaison du sol.

5. Accessoire orthopédique selon la revendication 4, **caractérisé en ce que** la position de point zéro est susceptible d'être déterminée en fonction d'une hauteur actuelle du talon d'une chaussure utilisée avec l'accessoire.

6. Accessoire orthopédique selon l'une des revendications 1 à 5, **caractérisé en ce que** les parties de l'accessoire sont réalisées principalement rigides, et **en ce que** la commande d'au moins une majeure partie du déroulement du mouvement a lieu à l'intérieur d'un cycle de mouvement sans être influencée par des élasticités des matériaux grâce à la commande des résistances à l'écoulement.

7. Accessoire orthopédique selon l'une des revendications 1 à 6, **caractérisé en ce que** l'articulation rotative est une articulation de cheville.

8. Procédé pour la commande d'un accessoire orthopédique selon l'une des revendications 1 à 7, **caractérisé en ce que** l'on détermine une position de point zéro du mouvement rotatif, à partir de laquelle on détermine, dans un déroulement cyclique des mouvements, la résistance au mouvement pour au moins un sens de déplacement.

9. Procédé selon la revendication 8, **caractérisé en ce que** l'accessoire est arrêté au point zéro pour une exigence d'emploi définie par réglage de résistances au mouvement plus élevées dans les deux sens de déplacement.

10. Procédé selon la revendication 7 ou 8, appliqué à un accessoire orthopédique pour une extrémité inférieure, dans lequel on utilise un cycle de marche à titre de cycle de mouvement susceptible d'être commandé.
